# EUROPEAN PATENT SPECIFICATION

(11) **EP 1 718 269 B1**
(45) Date of publication and mention of the grant of the patent: **17.12.2014**
(21) Application number: 05724030.1
(22) Date of filing: 25.02.2005
(51) Int. Cl.: A61K 8/25, A61K 8/27, A61K 8/24, A61Q 1/02, A61K 8/58, A61K 8/891

(54) **MAKE-UP COMPOSITION**
MAKEUP-ZUSAMMENSETZUNG
COMPOSITION DE MAQUILLAGE

(30) Priority: 26.02.2004 US 548142 P; 15.09.2004 US 610096 P
(43) Date of publication of application: 08.11.2006
(73) Proprietor: The Procter & Gamble Company, Cincinnati, OH 45202 (US)
(72) Inventor: TANIGUCHI, Toshiya, Kobe, Hyogo 658-0032 (JP)
(74) Representative: Engisch, Gautier
(86) International application number: PCT/US2005/006399
(87) International publication number: WO 2005/082320

(56) References cited:
- WO-A-01/91704
- WO-A-03/015727
- US-A- 5 362 482
- US-A1- 2002 031 534
- DATABASE WPI Section Ch, Week 199138 Derwent Publications Ltd., London, GB; Class A96, AN 1991-276704 XP002222302 & JP 03 181411 A (YG MIYOSHI KASEI) 7 August 1991 (1991-08-07)

## Description

### FIELD OF THE INVENTION

The present invention relates to a make-up composition comprising a sebum solidifying powder and an inorganic oil absorbing powder for improved sebum control. The compositions of the present invention are particularly useful for foundation products in various product forms.

### BACKGROUND

A foundation composition can be applied to the face and other parts of the body to even skin tone and texture and to hide pores, imperfections, fine lines and the like. A foundation composition is also applied to moisturize the skin, to balance the oil level of the skin, and to provide protection against the adverse effects of sunlight, wind, and other environmental factors.

Foundation compositions are generally available in the form of liquid or cream suspensions, emulsions, gels, pressed powders or anhydrous oil and wax compositions. Emulsion-type foundations in the form of liquid are suitable in that they provide moisturizing effects by the water and water-soluble skin treatment agents incorporated. Foundation compositions in the form of solid, yet water-in-oil emulsion are also suitable. Such solid emulsion foundations aim to address the drawbacks of conventional liquid form foundations and solid foundations. These foundations can be filled in a wide variety of packaging, including compacts, and is increasing popularity among consumers. References which disclose such foundation compositions include Japanese patent publications A-2-88511, A-3-261707, A-7-267819, A-11-209243, US patent 5,362,482, and PCT publication WO 01/91704.

Recently, consumers have become to seek foundation products that have sebum control benefit, while also having moisturizing benefits. Powders having sebum control benefit are known. Balancing of sebum control benefit and moisturizing benefit is yet challenging. In solid and liquid emulsion forms, incorporation of a meaningful amount of effective sebum controlling powders is difficult due to the limited amount of total powders that can be incorporated in the composition. Further, decreasing of other components may affect the spreadability of the composition. In powder forms, incorporation of a large amount of effective sebum controlling powders may be technically feasible, however, such may lead to a composition providing undesirable dry and tight feel to the skin.

Based on the foregoing, there is a need for a make-up composition which provides improved sebum control benefit, yet also provides good spreadability and moisturization to the skin and leaves the skin with a fresh and light feel.

None of the existing art provides all of the advantages and benefits of the present invention.

### SUMMARY OF THE INVENTION

The present invention is directed to a make-up composition comprising by weight: from 1% to 20% of a sebum solidifying powder comprising a base substance having a hydroxyapatite coated on the base substance, and a zinc oxide fixed to the coating layer of the hydroxyapatite, wherein the zinc oxide is from 15% to 25% by weight of the sebum solidifying powder;
from 1% to 10% of an inorganic oil absorbing powder having an oil absorbency of at least 200mℓ/100g wherein the weight ratio of the sebum solidifying powder to the inorganic oil absorbing powder is from 1:1.5 to 20:1; and
a suitable carrier.

By formulating powders of certain characteristics at an appropriate amount and ratio, a make-up composition which provides improved sebum control benefit, yet also provides good spreadability and moisturization to the skin and leaves the skin with a fresh and light feel is obtained.

These and other features, aspects, and advantages of the present invention will become evident to those skilled in the art from a reading of the present disclosure with the appended claims.

### DETAILED DESCRIPTION OF THE INVENTION

While the specification concludes with claims particularly pointing out and distinctly claiming the invention, it is believed that the present invention will be better understood from the following description.

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

All ingredients such as actives and other ingredients useful herein may be categorized or described by their cosmetic and/or therapeutic benefit or their postulated mode of action. However, it is to be understood that the active and other ingredients useful herein can, in some instances, provide more than one cosmetic and/or therapeutic benefit or operate via more than one mode of action. Therefore, classifications herein are made for the sake of convenience and are not intended to limit an ingredient to the particularly stated application or applications listed.

### SEBUM SOLIDIFYING POWDER

The present composition comprises from 1% to 20%, of a sebum solidifying powder comprising a base substance having a hydroxyapatite coated on the base substance, and a zinc oxide fixed to the coating layer of the hydroxyapatite, wherein the zinc oxide is from 15% to 25% by weight of the sebum solidifying powder. When incorporated in solid and liquid water-in-oil emulsion forms, the composition comprises from 1% to 15%, preferably from 2% to 7%, of a sebum solidifying powder.

The sebum solidifying powders useful herein are those having a platelet-like shape, and coated with low crystalline zinc oxide, amorphous zinc oxide, or mixtures thereof. The ratio of zinc oxide to the powder is important for providing sebum solidifying effect. The base substance may be any organic or inorganic substances that are useful for cosmetic use, including those listed below under "Powder Component". The sebum solidifying powder herein can be suitably made according to the methods disclosed in US 2002/0031534 A1. The sebum solidifying powders may be surface treated. The sebum solidifying powders useful herein have the ability to solidify sebum, i.e., are effective in adsorbing free fatty acid, diglyceride, and triglyceride, and solidifying them by forming zinc salts thereof, such that a film is formed within about 30 minutes. Moreover, the originally glossy sebum changes appearance into a matte film. Such capability can be distinguished from other oil absorbing powders, which are not selective in the type of oil to the absorbed, and do not form a film after absorbing an oil, thus may leave glossy gels and pastes after absorbing the sebum. Change in appearance provides a noticeable signal to the user that sebum has been controlled. Sebum solidifying effect may be conveniently measured by mixing a certain amount of powder with a certain amount of artificial sebum, mixing for a certain period of time, and allowing to stand until solidified or showing matte appearance. The time taken for the mixture to solidify or to change appearance is recorded. The shorter the time taken to solidify or change appearance, the higher the solidifying effect is of the powder.

It has been surprisingly found that, when combined with the later described inorganic oil absorbing powder, the sebum solidifying effect of the sebum solidifying powder is enhanced. Namely, the combined use of a certain total weight of sebum solidifying powder and inorganic oil absorbing powder provides improved sebum solidifying effect compared to use of the same total weight of sebum solidifying powder only. Such is unexpected, because it is understood that oil absorbing powders do not necessarily contribute in solidifying sebum. What is also unexpected is that such enhancing effect is seen more significantly in inorganic oil absorbing powders.

Thus, the present composition provides improved sebum control benefit despite the level of sebum controlling powders is relatively low. By keeping the level of sebum controlling powders low, the entire composition maintains flexibility to accommodate other components which provide basic functions of a make-up composition. Such is particularly true for the water-in-oil compositions herein. In water-in-oil compositions, the total amount of powder component is preferably kept low. By keeping the level of powder component low, the entire composition maintains flexibility to accommodate other components which provide spreadability, moisturization, and fresh and light feel.

Commercially available spherical oil absorbing powders useful herein include mica coated with hydroxyapatite, 20% zinc oxide with tradename PLV-20, and the same powder surface treated with Methicone with tradename SI-PLV-20, both available from Miyoshi Kasei, Inc.

### INORGANIC OIL ABSORBING POWDER

The present composition comprises from 1% to 10%, preferably from 2% to 7%, of an inorganic oil absorbing powder having an oil absorbency of at least 200mℓ/100g, wherein the weight ratio of the sebum solidifying powder to the inorganic oil absorbing powder is from 1:1.5 to 20:1, preferably from 1:1 to 2:1. Oil absorbency is a unit well known to the artisan, and which can be measured via: JIS K5101 No.21 "Test Method for Oil Absorbency Level".

Inorganic absorbing powders useful herein include silica, silicate salts, carbonate salts, metal oxides, and hydroxyapatite. Both untreated and surface treated powders can be used, depending on the compatibility with the remainder of the composition. Particularly useful herein are spherical silica powders having an average particle size of from 1µm to 30µm.

Commercially available inorganic oil absorbing powders useful herein include spherical silica with tradename SILDEX H-52 available from Asahi Glass Company Co., Ltd, the same surface treated and with tradename SI SILDEX H-52 available from Miyoshi Kasei, Inc., and spherical silica with tradename GODD BALL SF-16C available from Suzuki Yushi Industrial Co. Ltd., all having an oil absorbency of more than 200mℓ/100g.

### SUITABLE CARRIER AND COMPOSITION FORMS

The combination of sebum solidifying powder and inorganic oil absorbing powder of the present invention can be incorporated in various make-up compositions for providing sebum control, while minimizing the affect to other benefits of the composition. For example, the combination may be formulated in a powder carrier for making a loose powder product form. Other composition forms and their respective suitable carriers are listed hereinbelow.

Water-in-oil emulsified make-up composition in solid form comprising by weight:
(a) from 10% to 25% of a powder component; the powder component comprising by weight of the entire composition:
   (i) from 1% to 15% of the sebum solidifying powder; and
   (ii) from 1 % to 10% of the inorganic oil absorbing powder;
(b) from 20% to 50% of a volatile silicone oil;
(c) from 0.5% to 10% of a non-volatile oil;
(d) from 1% to 5% of a lipophilic surfactant having an HLB of less than 8;
(e) water in an amount such that the total of the volatile silicone oil and water is at least 50%; and
(f) from 1% to 5% of a solid wax.

Water-in-oil emulsified make-up composition in liquid form comprising by weight:
(a) from 10% to 25% of a powder component; the powder component comprising by weight of the entire composition:
   (i) from 1% to 15% of the sebum solidifying powder;
   (ii) from 1% to 10% of the inorganic oil absorbing powder;
(b) from 20% to 50% of a volatile silicone oil;
(c) from 0.5% to 20% of a non-volatile oil;
(d) from 0.1 % to 10% of a lipophilic surfactant having an HLB of less than 8;
(e) from 10% to 60% of water; and
(f) from 0% to 5% of optional thickener.

Composition in powder form comprising by weight:
(a) from 75% to 97% of a powder component; the powder component comprising by weight of the entire composition:
   (i) from 1% to 20% of the sebum solidifying powder; and
   (ii) from 1% to 10% of the inorganic oil absorbing powder;
(b) from 3% to 25% of a binder selected from the group consisting of volatile silicone oil, non-volatile oil, thickener, lipophilic surfactant, water, humectant, and mixtures thereof.

### POWDER COMPONENT

The composition of the present invention comprises a powder component. The powders included in the powder component herein are typically hydrophobic in nature, or hydrophobically treated. The species and levels of the powders other than those described above are selected to provide, for example, shade, coverage, UV protection benefit, good wear performance, and stability in the composition. Depending on the needs of the product, colorless powders may be selected for providing a colorless foundation, and/or a make up base composition.

Other powders useful for the powder component herein are clay mineral powders such as talc, mica, sericite, silica, magnesium silicate, synthetic fluorphlogopite, calcium silicate, aluminum silicate, bentonite and montmorillonite; pearl powders such as alumina, barium sulfate, calcium secondary phosphate, calcium carbonate, titanium oxide, finely divided titanium oxide, zirconium oxide, zinc oxide, hydroxy apatite, iron oxide, iron titanate, ultramarine blue, Prussian blue, chromium oxide, chromium hydroxide, cobalt oxide, cobalt titanate, titanium oxide coated mica; organic powders such as polyester, polyethylene, polystyrene, methyl methacrylate resin, cellulose, 12-nylon, 6-nylon, styrene-acrylic acid copolymers, polypropylene, vinyl chloride polymer, tetrafluoroethylene polymer, boron nitride, fish scale guanine, laked tar color dyes, and laked natural color dyes. Such powders may be treated with a hydrophobical treatment agent, including: silicone such as Methicone, Dimethicone and perfluoroalkylsilane; fatty material such as stearic acid; metal soap such as aluminium dimyristate; aluminium hydrogenated tallow glutamate, hydrogenated lecithin, lauroyl lysine, aluminium salt of perfluoroalkyl phosphate, and mixtures thereof.

Soft focus powders are also useful herein. What is meant by soft focus powder is a powder that is particularly effective in providing a soft focus effect to the composition, namely natural finish yet having good coverage for minimizing the appearance of skin troubles, when incorporated in the defined amount. Specifically, the soft focus powder herein must meet two parameters for providing such an effect. First, both the Total Luminous Transmittance (Tt) and Diffuse Luminous Transmittance (Td) of the powder are relatively high. The spherical soft focus powders of the present invention have a Total Luminous Transmittance (Tt) of from about 55 to about 90 and a Diffuse Luminous Transmittance (Td) of from about 34 to about 81. These powders are typically spherical. Without being bound by theory, it is believed that, by having such high values, the spherical soft focus powder exhibits a high transparency, thereby providing an overall natural finish. Second, the spherical soft focus powders of the present invention have a relatively high Haze value {(Td / Tt) x 100} of from about 62 to about 90. Without being bound by theory, it is believed that, by having such high Haze value, the contrast between lighted area of the skin and shaded area of the skin (such as pores and wrinkles) is minimized for reducing the appearance of the trouble areas. The use of the soft focus powder herein having such high Total Luminous Transmittance (Tt) and Diffuse Luminous Transmittance (Td), and high Haze value {(Td / Tt) x 100} is particularly effective for the present composition which contains a relatively low level of total powder component. These powders are typically spherical.

Total Luminous Transmittance (Tt), Diffuse Luminous Transmittance (Td), and Haze value {(Td / Tt) x 100} can be measured and calculated by the artisan by reference to ASTM D 1003-00 "Standard Test Method for Haze and Luminous Transmittance of Transparent Plastics". Although the powders herein are not plastics, the same principles of this specific standard test can be applied.

Spherical soft focus powders useful herein include spherical alumina, such as those commercially available with tradename SA-Alumina Beads, available from Miyoshi Kasei Inc., having a Total Luminous Transmittance (Tt) of 62-72, Diffuse Luminous Transmittance (Td) of 45-55, and Haze value {(Td / Tt) x 100} of 70-80.

Organic oil absorbing powders are useful herein, such as silicone elastomers, and methyl methacrylate copolymers. Commercially available powders useful herein include vinyl dimethicone/methicone silsesquioxane crosspolymer with tradename KSP-100 and KSP-101 available from ShinEtsu Chemical, hardened polyorgano siloxane elastomers with tradename TREFIL E-506C available from Dow Corning, and methyl methacrylate copolymer with tradename SA-GMP-0820 available from GANZ Chemical Co., Ltd. surface treated by Miyoshi Kasei, Inc.

Spherical powders other than the soft focus powders and oil absorbing powders may also be used. Unlimited examples of materials useful for making the spherical powders are; polyacrylates, silicates, sulfates, metal dioxides, carbonates, celluloses, polyalkylenes, vinyl acetates, polystyrenes, polyamides, acrylic acid ethers, silicones, and mixtures and complexes thereof. Specifically, materials useful herein include polyacrylates such as nylon, silicates such as calcium silicate, magnesium silicate, barium silicate, aluminium silicate and silica beads; metal dioxides such as titanium dioxide and aluminium hydroxide; carbonates such as calcium carbonate, magnesium carbonate; celluloses; polyalkylenes such as polyethylene, and polypropylene; vinyl acetates; polystyrenes; polyamides; acrylic acid ethers such as acrylic acid methyl ether and acrylic acid ethyl ether; polyvinyl pyrrolidones; and silicones such as polyorganosilsesquioxane resin.

Commercially available spherical powders highly useful herein include Nylon-12 with tradename NYLON POWDER series available from Toray.

### VOLATILE SILICONE OIL

Useful for the present invention is a volatile silicone oil. When incorporated in water-in-oil emulsions, preferably, the amount of the volatile silicone oil is controlled so that the composition comprises from 20% to 50% of the volatile silicone oil, and the total of the volatile silicone oil and water is more than 50% of the entire composition. Without being bound by theory, the species and levels of the volatile silicone oil herein is believed to provide improved refreshing and light feeling to the skin, without necessarily leaving a dried feeling to the skin. Volatile silicone oils can also be used as a binder for powder forms of the present composition.

The volatile silicone oil useful herein are selected from those having a boiling point of from 60 to 260°C, preferably those having from 2 to 7 silicon atoms.
The volatile silicone oils useful herein include polyalkyl or polyaryl siloxanes with the following structure (I): 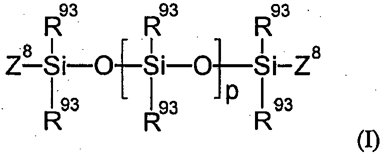 wherein R⁹³ is independently alkyl or aryl, and p is an integer from about 0 to about 5. Z⁸ represents groups which block the ends of the silicone chains. Preferably, R⁹³ groups include methyl, ethyl, propyl, phenyl, methylphenyl and phenylmethyl, Z⁸ groups include hydroxy, methyl, methoxy, ethoxy, propoxy, and aryloxy. More preferably, R⁹³ groups and Z⁸ groups are methyl groups. The preferred volatile silicone compounds are hexamethyldisiloxane, octamethyltrisiloxane, decamethyltetrasiloxane, hexadecamethylheptasiloxane. Commercially available volatile silicone compounds useful herein include octamethyltrisiloxane with tradename SH200C-1cs, decamethyltetrasiloxane with tradename SH200C-1.5cs, hexadecamethylheptasiloxane with tradename SH200C-2cs, all available from Dow Coming.

The volatile silicone oils useful herein also include a cyclic silicone compound having the formula: 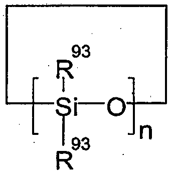 wherein R⁹³ is independently alkyl or aryl, and n is an integer of from 3 to 7.

Preferably, R⁹³ groups include methyl, ethyl, propyl, phenyl, methylphenyl and phenylmethyl. More preferably, R⁹³ groups are methyl groups. The preferred volatile silicone compounds are octamethylcyclotetrasiloxane, decamethylcyclopentasiloxane, tetradecamethylcyclohexasiloxane. Commercially available volatile silicone compounds useful herein include octamethylcyclotetrasiloxane with tradename SH244, decamethylcyclopentasiloxane with tradename DC245 and SH245, and dodeamethylcyclohexasiloxane with tradename DC246; all available from Dow Corning.

### NON-VOLATILE OIL

Useful for the composition of the present invention comprises a non-volatile oil. When incorporated in water-in-oil emulsions, preferably, the amount is from 0.5% to 20%. When the emulsion is made into solid form, preferably, the amount is from 0.5% to 10%. Without being bound by theory, the species and levels of the non-volatile oil herein is believed to provide improved smoothness to the skin, and also alleviate dry feeling of the skin. Non-volatile oils can also be used as a binder for powder forms of the present composition.

Non-volatile oils useful herein are, for example, tridecyl isononanoate, isostearyl isostearate, isocetyl isosteatrate, isopropyl isostearate, isodecyl isonoanoate, cetyl octanoate, isononyl isononanoate, diisopropyl myristate, isocetyl myristate, isotridecyl myristate, isopropyl myristate, isostearyl palmitate, isocetyl palmitate, isodecyl palmitate, isopropyl palmitate, octyl palmitate, caprylic/capric acid triglyceride, glyceryl tri-2-ethylhexanoate, neopentyl glycol di(2-ethyl hexanoate), diisopropyl dimerate, tocopherol, tocopherol acetate, avocado oil, camellia oil, turtle oil, macadamia nut oil, corn oil, mink oil, olive oil, rapeseed oil, eggyolk oil, sesame oil, persic oil, wheat germ oil, pasanqua oil, castor oil, linseed oil, safflower oil, cotton seed oil, perillic oil, soybean oil, peanut oil, tea seed oil, kaya oil, rice bran oil, china paulownia oil, Japanese paulownia oil, jojoba oil, rice germ oil, glycerol trioctanate, glycerol triisopalmiatate, trimethylolpropane triisostearate, isopropyl myristate, glycerol tri-2-ethylhexanoate, pentaerythritol tetra-2-ethylhexanoate, lanolin, liquid lanolin, liquid paraffin, squalane, vaseline, and mixtures thereof. Commercially available oils include, for example, tridecyl isononanoate with tradename Crodamol TN available from Croda, Hexalan available from Nisshin Seiyu, and tocopherol acetates available from Eisai.

Non-volatile oils useful herein also include polyalkyl or polyaryl siloxanes with the following structure (I) 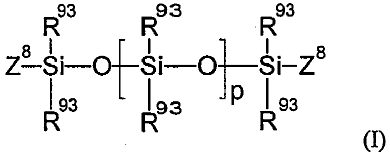 wherein R⁹³ is alkyl or aryl, and p is an integer from 7 to 8,000. Z⁸ represents groups which block the ends of the silicone chains. The alkyl or aryl groups substituted on the siloxane chain (R⁹³) or at the ends of the siloxane chains Z⁸ can have any structure as long as the resulting silicone remains fluid at room temperature, is dispersible, is neither irritating, toxic nor otherwise harmful when applied to the skin, is compatible with the other components of the composition, and is chemically stable under normal use and storage conditions. Suitable Z⁸ groups include hydroxy, methyl, methoxy, ethoxy, propoxy, and aryloxy. The two R⁹³ groups on the silicon atom may represent the same group or different groups. Preferably, the two R⁹³ groups represent the same group. Suitable R⁹³ groups include methyl, ethyl, propyl, phenyl, methylphenyl and phenylmethyl. The preferred silicone compounds are polydimethylsiloxane, polydiethylsiloxane, and polymethylphenylsiloxane. Polydimethylsiloxane, which is also known as dimethicone, is especially preferred. The polyalkylsiloxanes that can be used include, for example, polydimethylsiloxanes. These silicone compounds are available, for example, from the General Electric Company in their Viscasil^{®} and SF 96 series, and from Dow Corning in their Dow Corning 200 series.

Polyalkylaryl siloxane fluids can also be used and include, for example, polymethylphenylsiloxanes. These siloxanes are available, for example, from the General Electric Company as SF 1075 methyl phenyl fluid or from Dow Corning as 556 Cosmetic Grade Fluid.

Non-volatile oils also useful herein are the various grades of mineral oils. Mineral oils are liquid mixtures of hydrocarbons that are obtained from petroleum. Specific examples of suitable hydrocarbons include paraffin oil, mineral oil, dodecane, isododecane, hexadecane, isohexadecane, eicosene, isoeicosene, tridecane, tetradecane, polybutene, polyisobutene, and mixtures thereof.

### THICKENER

Useful for the present invention is a thickener. Thickeners can be used for adding viscosity to liquid water-in-oil form compositions, for solidifying solid water-in-oil form compositions, and as a binder for the powder form compositions of the present invention. When used in liquid forms, the thickener is kept to about 5% of the entire composition. Solid water-in-oil emulsion forms comprise thickeners, typically solid wax, for solidifying the composition.

The thickeners useful herein are selected from the group consisting of fatty compounds, solid wax, gelling agents, inorganic thickeners, silicone elastomers, and mixtures thereof. The amount and type of thickeners are selected according to the desired viscosity and characteristics of the product.

### Fatty Compounds

Fatty compounds useful herein include stearic acid, palmitic acid, stearyl alcohol, cetyl alcohol, behenyl alcohol, stearic acid, palmitic acid, the polyethylene glycol ether of stearyl alcohol or cetyl alcohol having an average of 1 to 5 ethylene oxide units, and mixtures thereof. Preferred fatty compounds are selected from stearyl alcohol, cetyl alcohol, behenyl alcohol, the polyethylene glycol ether of stearyl alcohol having an average of 2 ethylene oxide units (steareth-2), the polyethylene glycol ether of cetyl alcohol having an average of 2 ethylene oxide units, and mixtures thereof.

### Solid Wax

The composition of the present invention may comprise a solid wax. The solid water-in-oil emulsion compositions of the present invention preferably comprise, by weight of the entire composition, from 1% to 5% of solid wax. Without being bound by theory, the species and levels of the solid wax herein is believed to provide consistency to the composition and coverage to the skin, while not negatively contributing to the spreadability upon application to the skin, and fresh and light feel of the skin.

The solid waxes useful herein are paraffin wax, microcrystalline wax, ozokerite wax, ceresin wax, carnauba wax, candellila wax, eicosanyl behenate, and mixtures thereof. A mixture of waxes is preferably used.

Commercially available solid waxes useful herein include: Candelilla wax NC-1630 available from Cerarica Noda, Ozokerite wax SP-1021 available from Strahl & Pitsh, and Eicosanyl behenate available from Cas Chemical.

### Gelling Agents

The gelling agents useful as thickeners of the present invention include esters and amides of fatty acid gellants, hydroxy acids, hydroxy fatty acids, other amide gellants, and crystalline gellants.

N-acyl amino acid amides useful herein are prepared from glutamic acid, lysine, glutamine, aspartic acid and mixtures thereof. Particularly preferred are n-acyl glutamic acid amides corresponding to the following formula:

R2-NH-CO-(CH2)2-CH-(NH-CO-R1)-CO-NH-R2

wherein R1 is an aliphatic hydrocarbon radical having from 12 to 22 carbon atoms, and R2 is an aliphatic hydrocarbon radical having from 4 to 12 carbon atoms. Nonlimiting examples of these include n-lauroyl-L-glutamic acid dibutyl amide, n-stearoyl-L-glutamic acid diheptyl amide, and mixtures thereof. Most preferred is n-lauroyl-L-glutamic acid dibutyl amide, also referred to as dibutyl lauroyl glutamide. This material is commercially available with tradename Gelling agent GP-1 available from Ajinomoto.

Other gelling agents suitable for use in the compositions include 12-hydroxystearic acid, esters of 12-hydroxystearic acid, amides of 12-hydroxystearic acid and combinations thereof. These preferred gellants include those which correspond to the following formula:

R1-CO-(CH2)10-CH-(OH)-(CH2)5-CH3

wherein R1 is R2 or NR2R3; and R2 and R3 are hydrogen, or an alkyl, aryl, or arylalkyl radical which is branched linear or cyclic and has from 1 to 22 carbon atoms; preferably, from about 1 to about 18 carbon atoms. R2 and R3 may be either the same or different; however, at least one is preferably a hydrogen atom. Preferred among these gellants are those selected from the group consisting of 12-hydroxystearic acid, 12-hydroxystearic acid methyl ester, 12-hydroxystearic acid ethyl ester, 12-hydroxystearic acid stearyl ester, 12-hydroxystearic acid benzyl ester, 12-hydroxystearic acid amide, isopropyl amide of 12-hydroxystearic acid, butyl amide of 12-hydroxystearic acid, benzyl amide of 12-hydroxystearic acid, phenyl amide of 12-hydroxystearic acid, t-butyl amide of 12-hydroxystearic acid, cyclohexyl amide of 12-hydroxystearic acid, 1-adamantyl amide of 12-hydroxystearic acid, 2-adamantyl amide of 12-hydroxystearic acid, diisopropyl amide of 12-hydroxystearic acid, and mixtures thereof; even more preferably, 12-hydroxystearic acid, isopropyl amide of 12-hydroxystearic acid, and combinations thereof. Most preferred is 12-hydroxystearic acid.

Suitable amide gellants include disubstituted or branched monoamide gellants, monosubstituted or branched diamide gellants, triamide gellants, and combinations thereof, excluding the n-acyl amino acid derivatives selected from the group consisting of n-acyl amino acid amides, n-acyl amino acid esters prepared from glutamic acid, lysine, glutamine, apartic acid, and combinations thereof, and which are specifically disclosed in U.S. Patent 5,429,816.

Alkyl amides or di- and tri-basic carboxylic acids or anhydrides suitable for use in the composition include alkyl amides of citric acid, tricarballylic acid, aconitic acid, nitrilotriacetic acid, succinic acid and itaconic acid such as 1,2,3-propane tributylamide, 2-hydroxy-1,2,3-propane tributylamide, 1-propene-1,2,3-triotylamide, N,N',N"-tri(acetodecylamide)amine, 2-dodecyl-N,N'-dihexylsuccinamide, and 2 dodecyl-N,N'-dibutylsuccinamide. Preferred are alkyl amides of dicarboxylic acids such as di-amides of alkyl succinic acids, alkenyl succinic acids, alkyl succinic anhydrides and alkenyl succinic anhydrides, more preferably 2-dodecyl-N,N'-dibutylsuccinamide.

### Inorganic Thickeners

Inorganic thickeners useful herein include hectorite, bentonite, montmorillonite, and bentone clays which have been modified to be compatible with oil. Preferably, the modification is quaternization with an ammonium compound. Preferable inorganic thickeners include quaternary ammonium modified hectorite. Commercially available oil swelling clay materials include benzyldimethyl stearyl ammonium hectorite with tradename Bentone 38 available from Elementis.

### Silicone Elastomers

Suitable for use herein are silicone elastomers which can be emulsifying or non-emulsifying crosslinked siloxane elastomers or mixtures thereof. The term "non-emulsifying," as used herein, defines crosslinked organopolysiloxane elastomers from which polyoxyalkylene units are absent. The term "emulsifying," as used herein, means crosslinked organopolysiloxane elastomers having at least one polyoxyalkylene (e.g., polyoxyethylene or polyoxypropylene) unit. Non-emulsifying elastomers useful in the present invention are formed via crosslinking organohydroenpolysiloxanes with an alpha, omega-diene. Emulsifying elastomers herein include polyoxyalkylene modified elastomers formed via crosslinking from organohydrogenpolysiloxanes with polyoxyalkylene dienes or organohydrogenpolysiloxanes containing at least one polyether group crosslinked with an alpha, omega-diene. Emulsifying crosslinked organopolysiloxane elastomer can notably be chosen from the crosslinked polymers described in US Patents 5,412,004, 5,837,793, and 5,811,487. In addition, an emulsifying elastomer comprised of dimethicone copolyol crosspolymer (and dimethicone) is available from Shin Etsu under the tradename KSG-21.

Non-emulsifying elastomers are dimethicone/vinyl dimethicone crosspolymers. Such dimethicone/vinyl dimethicone crosspolymers are supplied by a variety of suppliers including Dow Corning (DC 9040 and DC 9041), General Electric (SFE 839), Shin Etsu (KSG-15, 16, 18 [dimethicone/phenyl vinyl dimethicone crosspolymer]), and Grant Industries (GRANSIL™ line of elastomers). Cross-linked organopolysiloxane elastomers useful in the present invention and processes for making them are further described in U.S. Patent 4,970,252, 5,760,116, and 5,654,362. Additional crosslinked organopolysiloxane elastomers useful in the present invention are disclosed in Japanese Patent Application JP 61-18708, assigned to Pola Kasei Kogyo KK. Commercially available elastomers preferred for use herein are Dow Corning's 9040 silicone elastomer blend, Shin Etsu's KSG-21, and mixtures thereof.

### LIPOPHILIC SULFACTANT

The composition of the present invention may comprise a lipophilic surfactant. When incorporated in water-in-oil emulsion compositions of the present invention, the amount included is preferably from 0.1% to 10%. When incorporated in solid water-in-oil emulsion forms, the amount included is preferably from 1% to 5%. Without being bound by theory, the species and levels of the lipophilic surfactant herein are believed to provide a stable water-in-oil emulsion in view of the other components of the present invention. Lipophilic surfactants can also be used as a binder for powder forms of the present composition. The lipophilic surfactant herein has an HLB value of less than 8.

The HLB value is a theoretical index value which describes the hydrophilicity-hydrophobicity balance of a specific compound. Generally, it is recognized that the HLB index ranges from 0 (very hydrophobic) to 40 (very hydrophilic). The HLB value of the lipophilic surfactants may be found in tables and charts known in the art, or may be calculated with the following general equation: HLB = 7 + (hydrophobic group values) + (hydrophilic group values). The HLB and methods for calculating the HLB of a compound are explained in detail in Surfactant Science Series, Vol. 1: Nonionic Surfactants", pp 606-13, M. J. Schick (Marcel Dekker Inc., New York, 1966).

The lipophilic surfactant can be an ester-type surfactant. Ester-type surfactants useful herein include: sorbitan monoisostearate, sorbitan diisostearate, sorbitan sesquiisostearate, sorbitan monooleate, sorbitan dioleate, sorbitan sesquioleate, glyceryl monoisostearate, glyceryl diiostearate, glyceryl sesquiisostearate, glyceryl monooleate, glyceryl dioleate, glyceryl sesquioleate, diglyceryl diisostearate, diglyceryl dioleate, diglycerin monoisostearyl ether, diglycerin diisostearyl ether, and mixtures thereof.

Commercially available ester-type surfactants are, for example, sorbitan isostearate having a tradename Crill 6 available from Croda, and sorbitan sesquioleate with tradename Arlacel 83 available from Kao Atras.

The lipophilic surfactant can be a silicone-type surfactant. Silicone-type surfactants useful herein are (i), (ii), (iii), and (iv) as shown below, and mixtures thereof.
(i) dimethicone copolyols having the formulation: 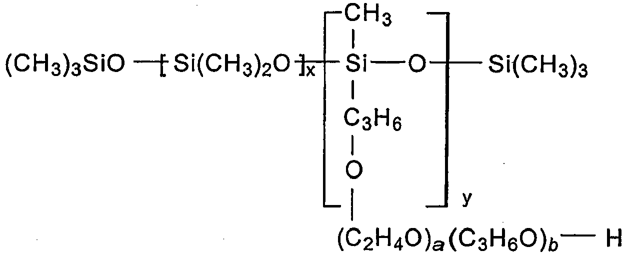 wherein x is an integer from 5 to 100, y is an integer from 1 to 50, a is zero or greater, b is zero or greater, the average sum of a+b being 1-100.
(ii) dimethicone copolyols having the formulation: 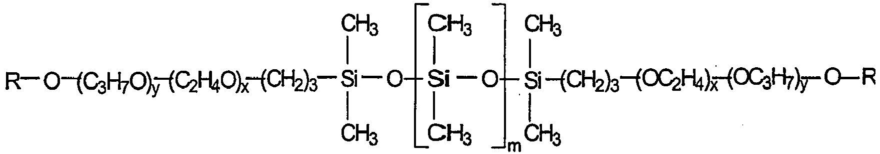 wherein R is selected from the group consisting of hydrogen, methyl, and combinations thereof, m is an integer from 5 to 100, x is independently zero or greater, y is independently zero or greater, the sum of x+y being 1-100.
(iii) branched polyether-polydiorganosiloxane emulsifiers herein having the formulation: 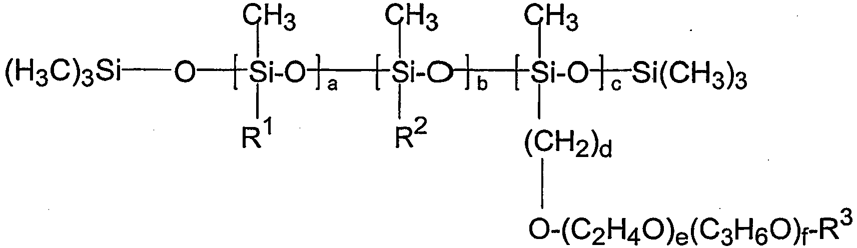 wherein R¹ is an alkyl group having from 1 to 20 carbons; R² is 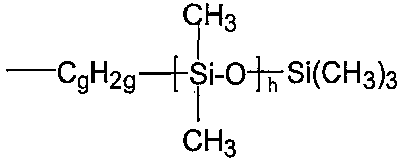 wherein g is from 1 to 5, and h is from 5 to 20; R³ is H or an alkyl group having from 1 to 5 carbons; e is from 5 to 20; f is from 0 to 10; a is from 20 to 100; b is from 1 to 15; c is from 1 to 15; and d is from 1 to 5.
(iv) alkyl dimethicone copolyols which are nonionic polysiloxane copolymer having emulsifying ability, comprising a methylpolysiloxane moiety, an alkyl methylpolysiloxane moiety, and a poly(oxyalkylene)methylpolysiloxane moiety; having an HLB from 4 to 6, and a molecular weight of from 10,000 to 20,000, wherein the alkyl group is made of from 10 to 22 carbons. Suitable alkyl dimethicone copolyols herein are those which have the following formulation: 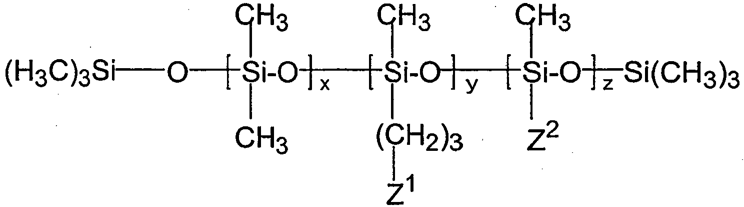 wherein Z¹ is O(C₂H₄O)ₚ(C₃H₆O)_{q}H, p is from 0 to 50, q is from 0 to 30, wherein p and q are not 0 at the same time; x is from 1 to 200, y is from 1 to 40, and z is from 1 to 100, and Z² is an alkyl group having from 10 to 22 carbons, preferably from 16 to 18 carbons.

Commercially available silicone-type surfactants are, for example, dimethicone copolyols DC5225C, BY22-012, BY22-008, SH3746M, SH3771M, SH3772M, SH3773M, SH3775M, SH3748, SH3749, and DC5200, all available from Dow Coming, and branched polyether-polydiorganosiloxane emulsifiers such as PEG-9 polydimethylsiloxyethyl Dimethicone, having an HLB of about 4 and a molecular weight of about 6,000 having a tradename KF 6028 available from ShinEtsu Chemical. Highly preferred alkyl dimethicone copolyols include cetyl dimethicone copolyol and stearyl dimethicone copolyol. A highly preferred commercially available alkyl dimethicone copolyol includes cetyl dimethicone copolyol, also called Methylpolysiloxane Cetylmethylpolysiloxane Poly(oxyethylene oxypropylene) Methylpolysiloxane Copolymer, having an HLB of 5 and a molecular weight of 13,000 having a tradename ABIL EM90 available from Goldschmidt Personal Care.

In a preferred embodiment, the lipophilic surfactant is a mixture of at least one ester-type surfactant and at least one silicone-type surfactant to provide a stable emulsion for the other essential components of the present invention.

### WATER

The water-in-oil emulsion compositions of the present invention comprise water in an amount sufficient to provide a discontinuous aqueous phase, preferably an amount such that the total of the volatile silicone oil and water is more than 50% of the entire composition. More preferably, the solid form compositions of the present invention comprise from 10% to 40% of water, and the liquid form compositions comprise from 10% to 60% of water. Without being bound by theory, the amount of water herein is believed to provide improved refreshing and light feeling to the skin, without necessarily leaving a dried feeling to the skin. Further, this amount of water allows the inclusion of optional water-soluble skin active agents as described below. Water can also be used as a binder for powder forms of the present composition.

In the present invention, deionized water is typically used. Water from natural sources including mineral cations can also be used, depending on the desired characteristic of the product.

### HUMECTANT

The composition of the present invention may further comprise a humectant. When incorporated in water-in-oil emulsions, preferably the amount is from 1% to 15%, more preferably from 2% to 7%. Humectants can also be used as a binder for powder forms of the present composition.

The humectants herein are selected from the group consisting of polyhydric alcohols, water soluble alkoxylated nonionic polymers, and mixtures thereof. Polyhydric alcohols useful herein include glycerin, propylene glycol, 1,3- butylene glycol, dipropylene glycol, diglycerin, sodium hyaluronate, and mixtures thereof.

Commercially available humectants herein include: glycerin available from Asahi Denka; propylene glycol with tradename LEXOL PG-865/855 available from Inolex, 1,2-PROPYLENE GLYCOL USP available from BASF; 1,3-butylene glycol available from Kyowa Hakko Kogyo; dipropylene glycol with the same tradename available from BASF; diglycerin with tradename DIGLYCEROL available from Solvay GmbH; sodium hyaluronate with tradename ACTIMOIST available from Active Organics, AVIAN SODIUM HYALURONATE series available from Intergen, HYALURONIC ACID Na available from Ichimaru Pharcos.

### FILM FORMING POLYMER

The compositions of the present invention may comprise a film forming polymer, for imparting wear and/or transfer resistant properties. When included, such materials are typically used in an amount of from 0.5% to 20%, preferably from 0.5% to 10% by weight, more preferably from 1% to 8%, by weight of the composition. Preferred polymers form a non-tacky film which is removable with water used with cleansers such as soap.

Examples of suitable film forming polymeric materials include:
a) sulfopolyester resins, such as AQ sulfopolyester resins, such as AQ29D, AQ35S, AQ38D, AQ38S, AQ48S, and AQ55S (available from Eastman Chemicals);
b) polyvinylacetate/polyvinyl alcohol polymers, such as Vinex resins available from Air Products, including Vinex 2034, Vinex 2144, and Vinex 2019;
c) acrylic resins, including water dispersible acrylic resins available from National Starch under the trade name "Dermacryl", including Dermacryl LT;
d) polyvinylpyrrolidones (PVP), including Luviskol K17, K30 and K90 (available from BASF), water soluble copolymers of PVP, including PVP/VA S-630 and W-735 and PVP/dimethylaminoethylmethacrylate Copolymers such as Copolymer 845 and Copolymer 937 available from ISP, as well as other PVP polymers disclosed by E.S. Barabas in the Encyclopedia of Polymer Science and Engineering, 2 Ed. Vol. 17 pp. 198-257;
e) high molecular weight silicones such as dimethicone and organic-substituted dimethicones, especially those with viscosities of greater than about 50,000 mPas;
f) high molecular weight hydrocarbon polymers with viscosities of greater than about 50,000 mPas;
g) organosiloxanes, including organosiloxane resins, fluid diorganopolysiloxane polymers and silicone ester waxes.

Examples of these polymers and cosmetic compositions containing them are found in PCT publication Nos. WO96/33689, published 10/31/96; WO97/17058, published 5/15/97; and US Patent No. 5,505,937 issued to Castrogiovanni et al. 4/9/96. Additional film forming polymers suitable for use herein include the water-insoluble polymer materials in aqueous emulsion and water soluble film forming polymers described in PCT publication No. WO98/18431, published 5/7/98. Examples of high molecular weight hydrocarbon polymers with viscosities of greater than about 50,000 mPas include polybutene, polybutene terephthalate, polydecene, polycyclopentadiene, and similar linear and branched high molecular weight hydrocarbons.

Preferred film forming polymers include organosiloxane resins comprising combinations of R₃SiO_{1/2} "M" units, R₂SiO "D" units, RSiO_{3/2} "T" units, SiO₂ "Q" units in ratios to each other that satisfy the relationship RₙSiO_{(4-n)/2} where n is a value between 1.0 and 1.50 and R is a methyl group. Note that a small amount, up to 5%, of silanol or alkoxy functionality may also be present in the resin structure as a result of processing. The organosiloxane resins must be solid at about 25°C and have a molecular weight range of from 1,000 to 10,000 grams/mole. The resin is soluble in organic solvents such as toluene, xylene, isoparaffins, and cyclosiloxanes or the volatile carrier, indicating that the resin is not sufficiently crosslinked such that the resin is insoluble in the volatile carrier. Particularly preferred are resins comprising repeating monofunctional or R₃SiO_{1/2} "M" units and the quadrofunctional or SiO₂ "Q" units, otherwise known as "MQ" resins as disclosed in U.S. Patent 5,330,747, Krzysik, issued July 19, 1994. In the present invention the ratio of the "M" to "Q" functional units is preferably about 0.7 and the value of n is 1.2. Organosiloxane resins such as these are commercially available such as Wacker 803 and 804 available from Wacker Silicones Corporation of Adrian Michigan, KP545 from Shin-Etsu Chemical and G. E. 1170-002 from the General Electric Company.

### SKIN ACTIVE AGENT

The compositions of the present invention may comprise a safe and effective amount of a skin active agent. The term "skin active agent" as used herein, means an active ingredient which provides a cosmetic and/or therapeutic effect to the area of application on the skin, hair, or nails. The skin active agents useful herein include skin lightening agents, anti-acne agents, emollients, non-steroidal anti-inflammatory agents, topical anaesthetics, artificial tanning agents, antiseptics, anti-microbial and anti-fungal actives, skin soothing agents, sunscreening agents, skin barrier repair agents, anti-wrinkle agents, anti-skin atrophy actives, lipids, sebum inhibitors, sebum inhibitors, skin sensates, protease inhibitors, skin tightening agents, anti-itch agents, hair growth inhibitors, desquamation enzyme enhancers, anti-glycation agents, and mixtures thereof. When included, the present composition comprises from 0.001% to 30%, preferably from 0.001% to 10% of at least one skin active agent.

The type and amount of skin active agents are selected so that the inclusion of a specific agent does not affect the stability of the composition.

Skin lightening agents useful herein refer to active ingredients that improve hyperpigmentation as compared to pre-treatment. Useful skin lightening agents herein include ascorbic acid compounds, vitamin B₃ compounds, azelaic acid, butyl hydroxyanisole, gallic acid and its derivatives, glycyrrhizinic acid, hydroquinone, kojic acid, arbutin, mulberry extract, and mixtures thereof. Use of combinations of skin lightening agents is believed to be advantageous in that they may provide skin lightening benefit through different mechanisms.

Ascorbic acid compounds useful herein include, ascorbic acid per se in the L-form, ascorbic acid salt, and derivatives thereof. Ascorbic acid salts useful herein include, sodium, potassium, lithium, calcium, magnesium, barium, ammonium and protamine salts. Ascorbic acid derivatives useful herein include, for example, esters of ascorbic acid, and ester salts of ascorbic acid. Particularly preferred ascorbic acid compounds include 2-o-D-glucopyranosyl-L-ascorbic acid, which is an ester of ascorbic acid and glucose and usually referred to as L-ascorbic acid 2-glucoside or ascorbyl glucoside, and its metal salts, and L-ascorbic acid phosphate ester salts such as sodium ascorbyl phosphate, potassium ascorbyl phosphate, magnesium ascorbyl phosphate, and calcium ascorbyl phosphate. Commercially available ascorbic compounds include magnesium ascorbyl phosphate available from Showa Denko, 2-o-D-glucopyranosyl-L-ascorbic acid available from Hayashibara and sodium L-ascorbyl phosphate with tradename STAY C available from Roche.

Vitamin B₃ compounds useful herein include, for example, those having the formula: 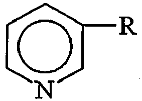 wherein R is -CONH₂ (e.g., niacinamide) or -CH₂OH (e.g., nicotinyl alcohol); derivatives thereof; and salts thereof. Exemplary derivatives of the foregoing vitamin B₃ compounds include nicotinic acid esters, including non-vasodilating esters of nicotinic acid, nicotinyl amino acids, nicotinyl alcohol esters of carboxylic acids, nicotinic acid N-oxide and niacinamide N-oxide. Preferred vitamin B₃ compounds are niacinamide and tocopherol nicotinate, and more preferred is niacinamide. In a preferred embodiment, the vitamin B₃ compound contains a limited amount of the salt form and is more preferably substantially free of salts of a vitamin B₃ compound. Preferably the vitamin B₃ compound contains less than 50% of such salt, and is more preferably essentially free of the salt form. Commercially available vitamin B₃ compounds that are highly useful herein include niacinamide USP available from Reilly.

Other hydrophobic skin lightening agents useful herein include ascorbic acid derivatives such as ascorbyl tetraisopalmitate (for example, VC-IP available from Nikko Chemical), ascorbyl palmitate (for example available from Roche Vitamins), ascorbyl dipalmitate (for example, NIKKOL CP available from Nikko Chemical); undecylenoyl phenyl alanine (for example, SEPIWHITE MSH available from Seppic); octadecenedioic acid (for example, ARLATONE DIOIC DCA available from Uniquema); oenothera biennis sead extract, and pyrus malus (apple) fruit extract, and mixtures thereof.

Other skin active agents useful herein include those selected from the group consisting of panthenol, tocopheryl nicotinate, benzoyl peroxide, 3-hydroxy benzoic acid, flavonoids (e.g., flavanone, chalcone), farnesol, phytantriol, glycolic acid, lactic acid, 4-hydroxy benzoic acid, acetyl salicylic acid, 2-hydroxybutanoic acid, 2-hydroxypentanoic acid, 2-hydroxyhexanoic acid, cis-retinoic acid, trans-retinoic acid, retinol, retinyl esters (e.g., retinyl propionate), phytic acid, N-acetyl-L-cysteine, lipoic acid, tocopherol and its esters (e.g., tocopheryl acetate), azelaic acid, arachidonic acid, tetracycline, ibuprofen, naproxen, ketoprofen, hydrocortisone, acetominophen, resorcinol, phenoxyethanol, phenoxypropanol, phenoxyisopropanol, 2,4,4'-trichloro-2'-hydroxy diphenyl ether, 3,4,4'-trichlorocarbanilide, octopirox, lidocaine hydrochloride, clotrimazole, miconazole, ketoconazole, neomycin sulfate, theophylline, and mixtures thereof.

### UV ABSORBING AGENT

The compositions of the present invention may comprise a safe and effective amount of a UV absorbing agent. A wide variety of conventional UV protecting agent are suitable for use herein, such as those decribed in U.S. Patent 5,087,445, Haffey et al, issued February 11, 1992; U.S. Patent 5,073,372, Turner et al, issued December 17,1991; U.S. Patent 5,073,371, Turner et al., issued December 17, 1991; and Segarin, et al, at Chapter VIII, pages 189 et seq., of Cosmetics Science and Technology (1972). When included, the present composition comprises from 0.5% to 20%, preferably from 1% to 15% of a UV absorbing agent.

UV absorbing agents useful herein are, for example, 2-ethylhexyl-p-methoxycinnamate (commercially available as PARSOL MCX), butylmethoxydibenzoyl-methane, 2-hydroxy-4-methoxybenzo-phenone, 2-phenylbenzimidazole-5-sulfonic acid, octyldimethyl-p-aminobenzoic acid, octocrylene, 2-ethylhexyl N,N-dimethyl-p-aminobenzoate, p-aminobenzoic acid, 2-phenylbenzimidazole-5-sulfonic acid, octocrylene, oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, Eusolex™ 6300, Octocrylene, Avobenzone (commercially available as Parsol 1789), and mixtures thereof.

### ADDITIONAL COMPONENTS

The compositions hereof may further contain additional components such as are conventionally used in topical products, e.g., for providing aesthetic or functional benefit to the composition or skin, such as sensory benefits relating to appearance, smell, or feel, therapeutic benefits, or prophylactic benefits (it is to be understood that the above-described required materials may themselves provide such benefits).

The CTFA Cosmetic Ingredient Handbook, Second Edition (1992) describes a wide variety of nonlimiting cosmetic and pharmaceutical ingredients commonly used in the industry, which are suitable for use in the topical compositions of the present invention. Such other materials may be dissolved or dispersed in the composition, depending on the relative solubilities of the components of the composition.

Examples of suitable topical ingredient classes include: anti-cellulite agents, antioxidants, radical scavengers, chelating agents, vitamins and derivatives thereof, abrasives, other oil absorbents, astringents, dyes, essential oils, fragrance, structuring agents, emulsifiers, solubilizing agents, anti-caking agents, antifoaming agents, binders, buffering agents, bulking agents, denaturants, pH adjusters, propellants, reducing agents, sequestrants, cosmetic biocides, alcohols and preservatives.

### PREPARATION OF THE COMPOSITION

The composition of the present invention may be made by a method well known in the art. In a suitable process, the solid and liquid water-in-oil emulsion compositions are made by the steps of:
1) dissolving the volatile silicone oil, non-volatile oil, lipophilic surfactant, slurry of powders dispersed in oil, and any other hydrophobic material in liquid form at ambient temperature in a sealed tank, to make a lipophilic mixture;
2) adding the powders into such lipophilic mixture and dispersing with a homogenizer at about 20-30°C;
3) separate from 1) and 2), heating and dissolving in water, humectants and any other hydrophilic material at about 75-80°C, and then cooling to about 20-30°C;
4) adding the product of step 3) to the product of step 2) to effect an emulsification;
5) heating and adding to the product of step 4), solid wax if present, and any remaining hydrophobic material at about 80-85°C; and
6) cooling the finally obtained emulsion to a temperature of about 60-80°C.
The obtained composition, which is still fluid at such temperature, is filled in an air-tight container and allowed to cool to room temperature typically using a cooling unit. The obtained compo sition is solid at ambient temperature, and thus can be poured into such container and left to solidify. The air-tight container is typically in a package form of a compact.

### EXAMPLES

The following examples further describe and demonstrate the preferred embodiments within the scope of the present invention. The examples are given solely for the purpose of illustration.

The following water-in-oil make-up compositions are formed by the process described herein:

**Composition for EXAMPLES 1-5**

| NO. | Components | Ex.1 | Ex.2 | Ex.3 | Ex.4 | Ex.5 |
|---|---|---|---|---|---|---|
| 1 | Cyclopentasiloxane *1 | 24.20 | 28.30 | 16.20 | 30.50 | 38.30 |
| 2 | PEG-9 Polydimethylsiloxyethyl Dimethicone *2 | 1.00 | 1.00 | 1.00 | 1.00 | 1.00 |
| 3 | Dimethicone and Dimethicone/Vinyl Dimethicone Crosspolymer *3 | - | 5.00 | - | - | - |
| 4 | Trimethylsiloxysilicate and Cyclopentasiloxane *4 | - | - | 5.00 | - | - |
| 5 | Tocopheryl Acetate *5 | 0.50 | 0.50 | 0.50 | 0.50 | 0.50 |
| 6 | Isotridecyl Isononanoate *6 | 1.00 | 1.00 | 1.00 | 1.00 | 1.00 |
| 7 | Sorbitan Monoisostearate *7 | 1.50 | 1.50 | 1.50 | 1.50 | 1.50 |
| 8 | Slurry of Iron Oxide and Cyclopentasiloxane and Dimethicone and Disodium Hydrogenated Glutamate *8 | 2.00 | 2.00 | 2.00 | 2.00 | - |
| 9 | Slurry of Titanium Dioxide and Cyclopentasiloxane and Dimethicone and Disodium Hydrogenated Glutamate *9 | 10.00 | - | 10.00 | - | - |
| 10 | Slurry of Titanium Dioxide and Cyclopentasiloxane and Dimethicone and Methicone *10 | 6.00 | - | 6.00 | - | 6.00 |
| 11 | Titanium Dioxide and Methicone * 11 | - | 8.00 | - | 6.00 | - |
| 12 | Alumina and Titanium Dioxide and Methicone *12 | - | 3.00 | - | 2.00 | - |
| 13 | Titanium Dioxide and Dimethicone and Aluminium Hydroxide and Stearic Acid *13 | - | 3.00 | - | 3.00 | - |
| 14 | Biconvex lens-shaped Methyl Methacryate Crosspolymer and Methicone *14 | - | - | - | 3.00 | - |
| 15 | Spherical Silica and Methicone *15 | 2.30 | 2.00 | 4.40 | 3.50 | 3.00 |
| 16 | Vinyl Dimethicone / Methicone Silsesquioxane Crosspolymer *16 | - | - | 1.00 | - | - |
| 17 | Mica and Zinc Oxide and Methicone and Hydroxyapatite *17 | 3.00 | 3.00 | 7.50 | 3.50 | 5.00 |
| 18 | Mica coated with Methicone *18 | 2.00 | - | 1.00 | 1.00 | - |
| 19 | Water | 35.00 | 30.00 | 30.00 | 30.00 | 30.00 |
| 20 | Polyvinylpyrrolidone *19 | - | - | 1.00 | - | - |
| 21 | Niacinamide *20 | 2.00 | 2.00 | 2.00 | 2.00 | 2.00 |
| 22 | Preservative | 0.45 | 0.45 | 0.45 | 0.45 | 0.45 |
| 23 | Panthenol *21 | 0.25 | 0.25 | 0.25 | 0.25 | 0.25 |
| 24 | Glycerin*22 | - | - | - | 1.00 | - |
| 25 | Butylene Glycol *23 | 5.00 | 5.00 | 5.00 | 4.00 | 5.00 |
| 26 | Candelilla Wax*24 | 2.00 | 2.00 | 2.00 | 2.00 | 4.00 |
| 27 | Ceresin *25 | 1.80 | 1.80 | 1.80 | 1.80 | - |
| 28 | Microcrystalline Wax *26 | - | - | - | - | 2.00 |
| Total | | 100.00 | 100.00 | 100.00 | 100.00 | 100.00 |

**Compositions for EXAMPLES 6-10**

| NO. | Components | Ex.6 | Ex.7 | Ex.8 | Ex.9 | Ex.10 |
|---|---|---|---|---|---|---|
| 1 | Cyclopentasiloxane *1 | 24.30 | 25.05 | 23.05 | 25.05 | 20.80 |
| 2 | PEG-9 Polydimethylsiloxyethyl Dimethicone *2 | 1.00 | - | 1.00 | - | 1.00 |
| 3 | Dimethicone and Dimethicone/Vinyl Dimethicone Crosspolymer *3 | 3.00 | 1.00 | 2.00 | 1.00 | - |
| 4 | Cethyl Dimethicone Copolyol *44 | - | 2.00 | - | 2.00 | 1.00 |
| 5 | Ethylhexyl Methoxycinnamate *42 | - | 4.00 | 4.00 | 4.00 | 4.00 |
| 6 | Trimethylsiloxysilicate and Cyclopentasiloxane *4 | - | - | - | - | 2.00 |
| 7 | Tocopheryl Acetate *5 | 0.50 | - | - | - | 0.50 |
| 8 | Isotridecyl Isononanoate *6 | 2.00 | 4.00 | 2.00 | 2.00 | 1.00 |
| 9 | Sorbitan Monoisostearate *7 | 1.50 | 1.00 | 1.00 | 1.00 | 1.00 |
| 10 | Slurry of Iron Oxide and Cyclopentasiloxane and Dimethicone and Disodium Hydrogenated Glutamate *8 | 2.00 | 2.00 | 2.00 | 2.00 | 2.00 |
| 11 | Slurry of Titanium Dioxide and Cyclopentasiloxane and Dimethicone and Disodium Hydrogenated Glutamate *9 | 8.00 | - | - | - | - |
| 12 | Slurry of Titanium Dioxide and Cyclopentasiloxane and Dimethicone and Methicone *10 | 6.00 | - | - | - | - |
| 13 | Titanium Dioxide and Methicone *11 | - | 8.00 | 8.00 | 7.00 | 8.00 |
| 14 | Alumina and Titanium Dioxide and Methicone *12 | - | - | - | 3.00 | - |
| 15 | Titanium Dioxide and Dimethicone and Aluminium Hydroxide and Stearic Acid *13 | - | 3.00 | 3.00 | 3.00 | 3.00 |
| 16 | Biconvex lens-shaped Methyl Methacryate Crosspolymer and Methicone *14 | - | - | - | - | 2.00 |
| 17 | Spherical Silica and Methicone *15 | 2.00 | 3.00 | 3.00 | 2.00 | 3.00 |
| 18 | Vinyl Dimethicone / Methicone Silsesquioxane Crosspolymer *16 | - | - | - | 0.50 | - |
| 19 | Mica and Zinc Oxide and Methicone and Hydroxyapatite *17 | 2.00 | 2.00 | 5.00 | 4.00 | 3.00 |
| 20 | Dimethicone and Titaneted Mica *45 | - | 0.50 | 0.50 | - | - |
| 21 | Mica coated with Methicone *18 | - | 1.00 | - | - | - |
| 22 | Water | 40.00 | 30.00 | 38.00 | 32.00 | 35.00 |
| 23 | Polyvinylpyrrolidone *19 | - | - | - | 1.00 | - |
| 24 | Niacinamide *20 | 2.00 | - | - | - | 2.00 |
| 25 | Preservative | 0.45 | 0.45 | 0.45 | 0.45 | 0.45 |
| 26 | Panthenol *21 | 0.25 | - | - | - | 0.25 |
| 27 | Glycerin *22 | 5.00 | - | - | 2.00 | - |
| 28 | Butylene Glycol *23 | - | 5.00 | 5.00 | 3.00 | 5.00 |
| 29 | Ethyl Alcohol | - | 8.00 | - | 5.00 | 5.00 |
| Total | | 100.00 | 100.00 | 100.00 | 100.00 | 100.00 |

**Compositions for Examples 11-15**

| **NO.** | **Component** | Ex. 11 | Ex. 12 | Ex. 13 | Ex. 14 | Ex. 15 |
|---|---|---|---|---|---|---|
| 1 | Polymethyl Methacrylate 20µm *27 | 3.00 | 1.00 | 2.00 | 10.00 | 20.00 |
| 2 | Polymethyl Methacrylate 8µm *28 | 8.00 | 3.00 | 6.00 | - | - |
| 3 | Polymethyl Methacrylate 6µm *29 | - | 6.00 | 12.00 | 5.00 | 20.00 |
| 4 | Nylon-12 5µm *30 | 2.00 | - | - | - | - |
| 5 | Talc coated with Methicone *31 | 14.59 | 32.10 | 18.10 | 16.60 | 17.10 |
| 6 | Mica coated with Methicone *18 | 10.00 | - | - | - | 5.00 |
| 7 | Spherical Silica and Methicone *15 | 5.00 | 3.00 | 5.00 | - | - |
| 8 | Spherical Silica *32 | - | - | - | 1.00 | 3.00 |
| 9 | Mica and Zinc Oxide and Methicone and Hydroxyapatite *17 | 5.00 | 6.00 | 8.00 | 10.00 | 5.00 |
| 10 | Sericite coated with Methicone *33 | 25.00 | 25.00 | 25.00 | 10.00 | - |
| 11 | Titanium Dioxide coated with Methicone *11 | 10.50 | 12.00 | 12.00 | 10.00 | 5.50 |
| 12 | Mica coated with Titanium Dioxide *34 | - | - | - | 25.00 | - |
| 13 | Methylparaben *35 | 0.30 | 0.30 | 0.30 | 0.30 | 0.30 |
| 14 | Propylparaben *36 | 0.10 | 0.10 | 0.10 | 0.10 | 0.10 |
| 15 | Iron Oxide coated with Methicone *37 | 2.00 | 2.50 | 2.50 | 2.00 | 1.00 |
| 16 | Methylphenyl Polysiloxane *38 | 8.50 | - | - | - | 7.90 |
| 17 | Dimethicone *39 | - | 4.80 | 4.00 | 5.90 | 10.00 |
| 18 | Dextrin Palmitate/Ethylhexanoate *40 | - | 0.10 | 0.50 | 0.10 | 0.10 |
| 19 | Sorbitan Monoisostearate *7 | 1.00 | - | - | - | - |
| 20 | D-delta-tocopherol *41 | 0.01 | - | - | - | - |
| 21 | Ethylhexyl Methoxycinnamate *42 | 5.00 | 4.00 | 4.00 | 4.00 | 4.00 |
| 22 | Cholesteryl 12-hydroxystearate *43 | - | 0.10 | 0.50 | - | - |
| 23 | Glycerin *22 | - | - | - | - | 1.00 |
| Total | | 100.00 | 100.00 | 100.00 | 100.00 | 100.00 |

### Definitions of Components

*1 Cyclopentasiloxane : SH245 available from Dow Corning
*2 PEG-9 Polydimethylsiloxyethyl Dimethicone : KF-6028 available from Shinetsu Silicone
*3 Dimethicone and Dimethicone/Vinyl Dimethicone Crosspolymer: KSG-16 available from Shinetsu Silicone
*4 Trimethylsiloxysilicate and Cyclopentasiloxane : Trimethylsiloxysilicate/Cyclomethicone D5 Blend available from GE Toshiba Silicones
*5 Tocopheryl Acetate : DL-a-Tocopheryl Acetate available from Eisai
*6 Isotridecyl Isononanoate : Crodamol TN available from Croda
*7 Sorbitan Monoisostearate : Crill 6 available from Croda
*8 Slurry of Iron Oxide and Cyclopentasiloxane and Dimethicone and Disodium Hydro genated Glutamate : SA/NAI-Y-10 / D5 (70%), SA/NAI-R-10 / D5 (65%) and SA/NAI-B-10 / D5 (75%) available from Miyoshi Kasei
*9 Slurry of Titanium Dioxide and Cyclopentasiloxane and Dimethicone and Disodium Hydrogenated Glutamate : SA/NAI-TR-10/D5(80%) available from Miyoshi Kasei
*10 Slurry of Titanium Dioxide and Cyclopentasiloxane and Dimethicone and Methicone: SAS-TTO-S-3/D5(50%) available from Miyoshi Kasei
*11 Titanium Dioxide and Methicone : SI Titanium Dioxide IS available from Miyoshi Kasei
*12 Alumina and Titanium Dioxide and Methicone : SI-LTSG30AFLAKEH(5%)LHC available Nippon Sheet Glass Co. Ltd. and surface treated by Miyoshi Kasei
*13 Titanium Dioxide and Dimethicone and Aluminum Hydroxide and Stearic Acid : SAST-UFTR-Z available from Miyoshi Kasei
*14 Biconvex lens-shaped Methyl Methacryate Crosspolymer and Methicone : SI-L-XC-F006Z available from Sekisui Plastics Co., Ltd. surface treated by Miyoshi Kasei
*15 Spherical Silica and Methicone : SI-SILDEX H-52 available from Asahi Glass Company Co., Ltd. and surface treated by Miyoshi Kasei, having an oil absorbency of more than 200 mℓ/100g
*16 Vinyl Dimethicone / Methicone Silsesquioxane Crosspolymer : KSP-100 available from Shinetsu Silicone
*17 Mica and Zinc Oxide and Methicone and Hydroxyapatite : SI-PLV-20 available from Miyoshi Kasei
*18 Mica coated with Methicone : SI Mica available from Miyoshi Kasei
*19 Polyvinylpyrrolidone : PVP K-30 available from BASF
*20 Niacinamide : Niacinamide available from Reilly Industries Inc.
*21 Panthenol : DL-Panthenol available from Alps Pharmaceutical Ind.
*22 Glycerin : Glycerin USP available from Asahi Denka
*23 Butylene Glycol: 1,3 Butylene Glycol available from Kyowa Hakko Kogyo
*24 Candelilla Wax : Candelilla wax NC-1630 available from Cerarica Noda
*25 Ceresin : Ozokerite wax SP-1021 available from Strahl & Pitsh
*26 Microcrystalline Wax: Multiwax 180M Yellow available from Witco Petroleum Specialties
*27 Polymethyl Methacrylate 20µm: GANZ PEARL GM-2000 available from GANZ CHEMICAL CO., LTD.
*28 Polymethyl Methacrylate 8µm: GANZ PEARL GM-0800S available from GANZ CHEMICAL CO., LTD.
*29 Polymethyl Methacrylate 6µm: GANZ PEARL GM-0600 available from GANZ CHEMICAL CO., LTD.
*30 Nylon-12 5µm: NYLON POWDER SP-500 available from TORAY
*31 Talc coated with Methicone: SI TALC available from MIYOSHI KASEI, INC.
*32 Spherical Silica: Godd ball SF-16C available from Suzuki Yushi Industrial Co., LTD. having an oil absorbency of more than 200 mℓ/100g
*33 Sericite coated with Methicone: SI SERICITE available from MIYOSHI KASEI, INC.
*34 Mica coated with Titanium Dioxide: FLAMENCO SUPER PEARL available from THE MEARL
*35 Methylparaben: METHYL PARABEN available from UENO PHARMACEUTICALS
*36 Propylparaben: PROPYL PARABEN available from UENO PHARMACEUTICALS
*37 Iron Oxide coated with Methicone: IRON OXIDE series available from DAITO KASEI KOUGYOU CO., LTD.
*38 Methylphenyl Polysiloxane: KF56 available from SHINETSU CHEMICAL CO., LTD.
*39 Dimethicone: SH200 available from Dow Corning
*40 Dextrin palmitate/ethylhexanoate: RHEOPEARL TT available from CHIBA FLOUR MILLING CO., LTD.
*41 D-delta-tocopherol: D-DELTA-TOCOPHEROL available from EISAI CO., LTD.
*42 Ethylhexyl Methoxycinnamate: PARSOL MCX available from ROCHE VITAMINS JAPAN K.K.
*43 Cholesteryl 12-hydroxystearate: SALACOS HS available from Nisshin Oil Mills
*44 Cetyl Dimethicone Copolyol: ABIL EM90 available from Goldschmidt Personal Care
*45 Dimethicone and Titaneted Mica: SA-FLAMENCO SUPER PEARL / SA-FLAMENCO RED available from MIYOSHI KASEI, INC.

### Method of Preparation

The make-up compositions of Examples 1 - 6 are prepared as follows:
1) Component numbers 1 through 10 are mixed with suitable mixer until homogeneous to make a lipophilic mixture.
2) Component numbers 11 through 18 are mixed with suitable mixer until homogeneous to make a powder mixture. The powder mixture is pulverized using pulverizer. The powder mixture is added into the lipophilic mixture with suitable mixer until homogeneous.
3) Component numbers 19 through 23 are dissolved with suitable mixer until all components are completely dissolved to make a water phase. The water phase is added into the product of step 2) to effect emulsion at room temperature using homogenizer.
4) Component number 26 through 28 are added into the product of step 3). For Examples 1 through 5, this is heated to dissolve at 80-85°C in a sealed tank for to melt the waxes.
5) Finally, the obtained emulsion is filled in an air-tight container and allowed to cool to room temperature using a cooling unit.

The make-up compositions of Examples 6-10 are prepared as follows:
1) Component numbers 1 through 12 are mixed with suitable mixer until homogeneous to make a lipophilic mixture.
2) Component numbers 13 through 19 are mixed with suitable mixer until homogeneous to make a powder mixture. The powder mixture is pulverized using pulverizer. The powder mixture and number 20, 21 are added into the lipophilic mixture with suitable mixer until homogeneous.
3) Component numbers 21 through 29 are dissolved with suitable mixer until all components are completely dissolved to make a water phase. The water phase is added into the product of step 2) to make emulsion at room temperature using disper type mixer or homogenizer.
4) Finally, the obtained emulsion is filled in an air-tight container.

The make-up compositions of Examples 11 - 15 are prepared as follows:
Component numbers 1-15 are mixed with a mixer to make a powder component. Separately, component numbers 20-23 are mixed with the aid of heat, component numbers 18 and 19 are added and mixed until dissolving well, and components 16 and 17 are add and mixed to make a binder component. The binder component is added into the powder component and mixed by a mixer. The obtained composition is pressed in a tray and set into a compact.

These embodiments represented by the previous examples have many advantages when applied to the skin as make up compositions. Examples 1-4 are useful as solid foundation products. Example 5 is useful as a make up base product. Examples 6 -10 are useful as a liquid foundation product. Examples 11-15 are useful as powder foundation products. All embodiment provide, for example, improved sebum control benefit to the skin without compromise to good skin coverage, and further provide improved spreadability and moisturization to the skin, and leaves the skin with a fresh and light feel.

## Claims

1. A make-up composition comprising by weight:
from 1% to 20% of a sebum solidifying powder comprising a base substance having a hydroxyapatite coated on the base substance, and a zinc oxide fixed to the coating layer of the hydroxyapatite, wherein the zinc oxide is from 15% to 25% by weight of the sebum solidifying powder;
from 1% to 10% of an inorganic oil absorbing powder having an oil absorbency of at least 200mℓ/100g wherein the weight ratio of the sebum solidifying powder to the inorganic oil absorbing powder is from 1:1.5 to 20:1; and
a suitable carrier.

2. The make-up composition according to Claim 1 wherein the inorganic oil absorbing powder is spherical silica having an average particle size of from 1 µm to 30µm.

3. The make-up composition according to Claim 1 wherein the weight ratio of the sebum solidifying powder to the inorganic oil absorbing powder is from 1:1 to 2:1.

4. The make-up composition of Claim 1, 2, or 3 wherein the composition is a water-in-oil emulsion composition comprising by weight:
(a) from 10% to 25% of a powder component; the powder component comprising by weight of the entire composition:
(i) from 1% to 15% of the sebum solidifying powder; and
(ii) from 1% to 10% of the inorganic oil absorbing powder;
(b) a volatile silicone oil;
(c) a non-volatile oil;
(d) a lipophilic surfactant having an HLB of less than 8; and
(e) water.

5. The water-in-oil emulsified make-up composition according to Claim 4 wherein the lipophilic surfactant comprises an ester-type surfactant and a silicone-type surfactant.

6. The water-in-oil emulsified make-up composition according to Claim 4 further comprising from 1% to 15% of a humectant.

7. The water-in-oil emulsified make-up composition according to Claim 4 further comprising a soft focus effect powder.

8. The water-in-oil emulsified make-up composition according to Claim 4 further comprising an organic oil absorbing powder.

9. The water-in-oil emulsified make-up composition according to Claim 4 further comprising a film forming polymer.

10. The water-in-oil emulsified make-up composition of Claim 4 wherein the composition is in solid form comprising by weight:
(b) from 20% to 50% of the volatile silicone oil;
(c) from 0.5% to 10% of the non-volatile oil;
(d) from 1% to 5% of the lipophilic surfactant;
(e) water in an amount such that the total of the volatile silicone oil and water is at least 50%; and
(f) from 1% to 5% of a solid wax.

11. The water-in-oil emulsified make-up composition of Claim 4 wherein the composition is in liquid form comprising by weight:
(b) from 20% to 50% of the volatile silicone oil;
(c) from 0.5% to 20% of the non-volatile oil;
(d) from 0.1 % to 10% of the lipophilic surfactant;
(e) from 10% to 60% of water;
(f) from 0% to 5% of optional thickener.

12. The make-up composition of Claim 1, 2, or 3 wherein the composition is in powder form comprising by weight:
(a) from 75% to 97% of a powder component; the powder component comprising by weight of the entire composition:
(i) from 1% to 20% of the sebum solidifying powder; and
(ii) from 1% to 10% of the inorganic oil absorbing powder; and
(b) from 3% to 25% of a binder selected from the group consisting of volatile silicone oil, non-volatile oil, thickener, lipophilic surfactant, water, humectant, and mixtures thereof.

## Patentansprüche

1. Make-up-Zusammensetzung, umfassend, bezogen auf das Gewicht: 1 % bis 20 % Sebum verfestigendes Pulver, das eine Basissubstanz mit einer Beschichtung aus Hydroxyapatit auf der Basissubstanz und ein auf der Beschichtung des Hydroxyapatits fixiertes Zinkoxid umfasst, wobei das Zinkoxid 15 Gew.-% bis 25 Gew.-% des Sebum verfestigenden Pulvers ausmacht; 1 % bis 10 % anorganisches Öl absorbierendes Pulver mit einer Ölabsorptionsfähigkeit von mindestens 200 ml / 100 g, wobei das Gewichtsverhältnis des Sebum verfestigenden Pulvers zum anorganisches Öl absorbierenden Pulver bei 1:1,5 bis 20:1 liegt; und einen geeigneten Träger.

2. Make-up-Zusammensetzung nach Anspruch 1, wobei das anorganisches Öl absorbierende Pulver sphärische Kieselsäure mit einer durchschnittlichen Teilchengröße von 1 µm bis 30 µm ist.

3. Make-up-Zusammensetzung nach Anspruch 1, wobei das Gewichtsverhältnis des Sebum verfestigenden Pulvers zum anorganisches Öl absorbierenden Pulver bei 1:1 bis 2:1 liegt.

4. Make-up-Zusammensetzung nach Anspruch 1, 2 oder 3, wobei die Zusammensetzung eine Wasser-in-Öl-Emulsionszusammensetzung ist, die, bezogen auf das Gewicht, umfasst:
(a) zu 10 % bis 25 % einer Pulverkomponente; wobei die Pulverkomponente, bezogen auf das Gewicht der gesamten Zusammensetzung, umfasst:
(i) zu 1 % bis 15 % das Sebum verfestigende Pulver; und
(ii) zu 1 % bis 10 % das anorganisches Öl absorbierende Pulver;
(b) ein flüchtiges Silikonöl;
(c) ein nicht-flüchtiges Öl;
(d) ein lipophiles Tensid mit einem HLB von unter 8; und
(e) Wasser.

5. Make-up-Zusammensetzung aus einer Wasser-in-Öl-Emulsion nach Anspruch 4, wobei das lipophile Tensid ein Tensid vom Ester-Typ und ein Tensid vom Silikon-Typ umfasst.

6. Make-up-Zusammensetzung aus einer Wasser-in-Öl-Emulsion nach Anspruch 4, ferner 1 % bis 15 % Befeuchtungsmittel umfassend.

7. Make-up-Zusammensetzung aus einer Wasser-in-Öl-Emulsion nach Anspruch 4, ferner ein Pulver mit Weichzeichnereffekt umfassend.

8. Make-up-Zusammensetzung aus einer Wasser-in-Öl-Emulsion nach Anspruch 4, ferner ein organisches Öl absorbierendes Pulver umfassend.

9. Make-up-Zusammensetzung aus einer Wasser-in-Öl-Emulsion nach Anspruch 4, ferner ein filmbildendes Polymer umfassend.

10. Make-up-Zusammensetzung aus einer Wasser-in-Öl-Emulsion nach Anspruch 4, wobei die Zusammensetzung in fester Form vorliegt und, bezogen auf das Gewicht, umfasst:
(b) zu 20 % bis 50 % das flüchtige Silikonöl;
(c) zu 0,5 % bis 10 % das nicht-flüchtige Öl;
(d) zu 1 % bis 5 % das lipophile Tensid;
(e) Wasser in einer solchen Menge, dass die Gesamtmenge des flüchtigen Silikonöls und des Wassers mindestens 50 % ausmacht; und
(f) zu 1 % bis 5 % ein festes Wachs.

11. Make-up-Zusammensetzung aus einer Wasser-in-Öl-Emulsion nach Anspruch 4, wobei die Zusammensetzung in flüssiger Form vorliegt und, bezogen auf das Gewicht, umfasst:
(b) zu 20 % bis 50 % das flüchtige Silikonöl;
(c) zu 0,5 % bis 20 % das nicht-flüchtige Öl;
(d) zu 0,1 % bis 10 % das lipophile Tensid;
(e) zu 10 % bis 60 % Wasser,
(f) zu 0 % bis 5 % optionales Verdickungsmittel.

12. Make-up-Zusammensetzung nach Anspruch 1, 2 oder 3, wobei die Zusammensetzung in Pulverform vorliegt und, bezogen auf das Gewicht, umfasst:
(a) zu 75 % bis 97 % eine Pulverkomponente; wobei die Pulverkomponente, bezogen auf das Gewicht der gesamten Zusammensetzung, umfasst:
(i) zu 1 % bis 20 % das Sebum verfestigende Pulver; und
(ii) zu 1 % bis 10 % das anorganisches Öl absorbierende Pulver; und
(b) zu 3 % bis 25 % ein Bindemittel, das ausgewählt ist aus der Gruppe bestehend aus flüchtigem Silikonöl, nicht-flüchtigem Öl, Verdickungsmittel, lipophilem Tensid, Wasser, Befeuchtungsmittel und Mischungen davon.

## Revendications

1. Composition de maquillage comprenant en poids : de 1 % à 20 % d'une poudre de solidification de sébum comprenant une substance de base possédant une hydroxyapatite revêtue sur la substance de base, et un oxyde de zinc fixé à la couche de revêtement de l'hydroxyapatite, dans laquelle l'oxyde de zinc va de 15 % à 25 % en poids de la poudre de solidification de sébum ; de 1 % à 10 % d'une poudre d'absorption d'huile inorganique possédant une absorbance d'huile d'au moins 200 mℓ/100 g, dans laquelle le rapport pondéral de la poudre de solidification de sébum sur la poudre d'absorption d'huile inorganique va de 1:1,5 à 20:1 ; et un véhicule approprié.

2. Composition de maquillage selon la revendication 1, dans laquelle la poudre d'absorption d'huile inorganique est de la silice sphérique possédant une taille moyenne de particules allant de 1 µm à 30 µm.

3. Composition de maquillage selon la revendication 1, dans laquelle le rapport pondéral de la poudre de solidification de sébum sur la poudre d'absorption d'huile inorganique va de 1:1 à 2:1.

4. Composition de maquillage selon la revendication 1, 2 ou 3, où la composition est une composition d'émulsion eau-dans-huile comprenant en poids :
(a) de 10 % à 25 % d'un composant en poudre ; le composant en poudre comprenant en poids de la composition entière :
(i) de 1 % à 15 % de la poudre de solidification de sébum ; et
(ii) de 1 % à 10 % de la poudre d'absorption d'huile inorganique ;
(b) une huile de silicone volatile ;
(c) une huile non volatile ;
(d) un agent tensioactif lipophile possédant un rapport hydrophile-lipophile inférieur à 8 ; et
(e) de l'eau.

5. Composition de maquillage en émulsion eau-dans-huile selon la revendication 4, dans laquelle l'agent tensioactif lipophile comprend un agent tensioactif de type ester et un agent tensioactif de type silicone.

6. Composition de maquillage en émulsion eau-dans-huile selon la revendication 4, comprenant en outre de 1 % à 15 % d'un humectant.

7. Composition de maquillage en émulsion eau-dans-huile selon la revendication 4, comprenant en outre une poudre à effet flou artistique.

8. Composition de maquillage en émulsion eau-dans-huile selon la revendication 4, comprenant en outre une poudre d'absorption d'huile organique.

9. Composition de maquillage en émulsion eau-dans-huile selon la revendication 4, comprenant en outre un polymère filmogène.

10. Composition de maquillage en émulsion eau-dans-huile selon la revendication 4, où la composition est sous forme solide comprenant en poids :
(b) de 20 % à 50 % de l'huile de silicone volatile ;
(c) de 0,5 % à 10 % de l'huile non volatile ;
(d) de 1 % à 5 % de l'agent tensioactif lipophile ;
(e) de l'eau en une quantité telle que le total de l'huile de silicone volatile et de l'eau est d'au moins 50 % ; et
(f) de 1 % à 5 % d'une cire solide.

11. Composition de maquillage en émulsion eau-dans-huile selon la revendication 4, où la composition est sous forme liquide comprenant en poids :
(b) de 20 % à 50 % de l'huile de silicone volatile ;
(c) de 0,5 % à 20 % de l'huile non volatile ;
(d) de 0,1 % à 10 % de l'agent tensioactif lipophile ;
(e) de 10 % à 60 % d'eau ;
(f) de 0 % à 5 % d'épaississant facultatif.

12. Composition de maquillage selon la revendication 1, 2 ou 3, où la composition est sous forme de poudre comprenant en poids :
(a) de 75 % à 97 % d'un composant en poudre ; le composant en poudre comprenant en poids de la composition entière :
(i) de 1 % à 20 % de la poudre de solidification de sébum ; et
(ii) de 1 % à 10 % de la poudre d'absorption d'huile inorganique ; et
(b) de 3 % à 25 % d'un liant choisi dans le groupe constitué d'huile de silicone volatile, huile non volatile, épaississant, agent tensioactif lipophile, eau, humectant, et leurs mélanges.
